(19) Europäisches Patentamt
European Patent Office
Office européen des brevets

(11) EP 1 839 646 A1

(12) **DEMANDE DE BREVET EUROPEEN**

(43) Date de publication:
**03.10.2007 Bulletin 2007/40**

(51) Int Cl.:
***A61K 8/49*** *(2006.01)* ***A61Q 5/06*** *(2006.01)*

(21) Numéro de dépôt: **07104860.7**

(22) Date de dépôt: **26.03.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **28.03.2006 FR 0651074**

(71) Demandeur: **L'Oréal**
**75008 Paris (FR)**

(72) Inventeurs:
- **Lagrange, Alain**
**77700, COUPVRAY (FR)**
- **Hercouet, Leïla**
**93360, NEUILLY PLAISANCE (FR)**

(74) Mandataire: **Wattremez, Catherine**
**L'OREAL - D.I.P.I.**
**25-29 Quai Aulagnier**
**92600 Asnières (FR)**

(54) **Composition tinctoriale contenant un colorant direct hydrazonique cationique particulier, procédé de teinture et dispositifs à plusieurs compartiments**

(57) La présente invention a pour objet une composition pour la teinture des fibres kératiniques humaines, comprenant :
(a) au moins un colorant cationique direct de formule (I), ou ses formes tautomères

$$[A\text{-}C(R_3)\text{=}N\text{-}N(R_1)\text{-}B]^+ X$$

dans laquelle :
$R_1$ et $R_3$ indépendamment l'un de l'autre, représentent un atome d'hydrogène ; un radical alkyle éventuellement porteur d'un groupement hydroxyle ;
A représente un hétérocycle cationique condensé ou non, éventuellement substitué, choisi parmi les cycles pyrimidinone, indole, benzothiazole ;
B représente un tricycle saturé ou insaturé, éventuellement substitué, choisi parmi les dibenzofuranes, les carbazoles, les anthraquinones ou les dibenzothiényles ;
X représente un anion ou un mélanges d'anions cosmétiquement acceptables ;

(b) au moins un additif cosmétique choisi parmi les agents épaississants et les tensio-actifs.

La présente invention concerne de même un procédé de coloration mettant en oeuvre ladite composition, en présence ou en l'absence d'un agent oxydant, ainsi qu'un dispositif à plusieurs compartiments.

EP 1 839 646 A1

## Description

L'invention a pour objet une composition tinctoriale pour la teinture des fibres kératiniques humaines et plus particulièrement des cheveux, comprenant un colorant direct hydrazonique cationique particulier, les procédés de teinture des fibres kératiniques humaines mettant en oeuvre une telle composition et un dispositif pour la mise en oeuvre de ces procédés.

Il est connu de teindre les fibres kératiniques humaines et en particulier les cheveux avec des compositions tinctoriales contenant des précurseurs de colorant d'oxydation, appelés généralement bases d'oxydation et coupleurs Ces bases d'oxydation et ces coupleurs sont des composés incolores ou faiblement colorés qui, associés à des agents oxydants tels que les peroxyde notamment le peroxyde d'hydrogène, donnent naissance par un processus de condensation oxydative à des composés colorés.

Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est-à-dire de la rendre plus visible.

Il est aussi connu de teindre les fibres kératiniques humaines par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Il est connu par exemple d'utiliser des colorants directs nitrés benzéniques, anthraquinoniques, nitropyridiniques, des colorants azoïques, xanthéniques, acridiniques aziniques ou des colorants triarylméthaniques.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur faible tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux. En outre, leur sensibilité à la lumière est dépendante de leur répartition uniforme ou en agrégats dans la fibre kératinique.

Les colorants directs peuvent être associés à des agents oxydants. Cependant, les colorants directs sont souvent sensibles à l'action des agents oxydants et des agents réducteurs, ce qui les rend généralement difficilement utilisables dans les compositions de teinture directe éclaircissante à base d'eau oxygénée et à base d'un agent alcalinisant ou dans des compositions de teinture d'oxydation en association avec des précurseurs du type bases d'oxydation ou coupleurs.

Il existe donc un réel besoin de rechercher des colorants directs chromatiques qui permettent de teindre les fibres kératiniques humaines aussi puissamment que les colorants d'oxydation, qui soient aussi stables qu'eux à la lumière, soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation à base de précurseurs de colorants d'oxydation les contenant.

Il existe aussi un réel besoin de rechercher des colorants directs qui permettent d'obtenir des montées de couleur comparables à celles obtenus avec les précurseurs de colorants d'oxydation.

En outre, il reste toujours essentiel d'améliorer encore l'innocuité des colorants, de rechercher des colorants ne dégradant pas la fibre kératinique et ayant une sélectivité diminuée par rapport aux colorants classiques.

Ces buts sont atteints avec la présente invention qui a pour objet une composition tinctoriale, comprenant dans un milieu approprié pour la teinture des fibres kératiniques humaines :

(a) au moins un colorant hydrazonique cationique direct de formule (I), ou ses formes tautomères :

$$[A\text{-}C(R_3)\text{=}N\text{-}N(R_1)\text{-}B]^+ X$$

dans laquelle :

$R_1$ et $R_3$ indépendamment l'un de l'autre, représentent un atome d'hydrogène ; un radical alkyle linéaire ou ramifié en $C_1$-$C_{10}$ éventuellement porteur d'un groupement hydroxyle, et de préférence représentent indépendamment l'un de l'autre un atome d'hydrogène ; un radical alkyle linéaire ou ramifié en $C_1$-$C_{10}$ ;

A représente un hétérocycle cationique condensé ou non, éventuellement substitué, choisi parmi les cycles pyrimidinone, indole, benzothiazole ; le cycle A étant relié au carbone de C(R3) par un atome de carbone ;
B représente un tricycle saturé ou insaturé, éventuellement substitué, choisi parmi les dibenzofuranes, les carbazoles, les anthraquinones ou les dibenzothiényles ; le tricycle, saturé ou insaturé, étant relié à l'atome d'azote de la fonction hydrazone par l'intermédiaire d'un atome de carbone de l'un des trois cycles ;
X représente un anion ou un mélanges d'anions cosmétiquement acceptables ;

(b) au moins un agent cosmétique choisi parmi les polymères épaississants et les tensio-actifs.

La présente invention a de même pour objet un procédé de coloration de fibres kératiniques humaines, notamment les cheveux, consistant à appliquer la composition décrite ci-dessus sur les fibres et à laisser poser pendant un temps suffisant pour obtenir la coloration souhaitée.

Elle concerne aussi un dispositif à plusieurs compartiments dans lequel au moins un premier compartiment renferme la composition tinctoriale comprenant le colorant de formule (I) au moins un deuxième compartiment renferme la composition oxydante.

L'invention a aussi pour objet l'utilisation d'un colorant de formule (I) tel que défini ci-dessus pour la teinture des fibres kératiniques.

Mais d'autres avantages et caractéristiques de la présente invention apparaîtront plus clairement à la lecture de la description et des exemples qui vont suivre.

Dans ce qui va suivre et à moins qu'une autre indication ne soit donnée, les bornes délimitant un domaine de valeurs sont comprises dans ce domaine de valeurs.

Selon un mode de réalisation particulier de l'invention, la composition comprend un colorant de formule (I) qui est tel que l'une des formes tautomères de ce colorant (I) présente un atome d'azote quaternisé engagé dans l'hétérocycle.

Plus particulièrement, ledit atome d'azote porte un substituant choisi parmi les radicaux alkyle linéaires ou ramifiés en $C_1$-$C_{10}$, de préférence en $C_1$-$C_6$ ; les radicaux hydroxyalkyle linéaires ou ramifiés en $C_1$-$C_{10}$, de préférence en $C_1$-$C_6$ ; les radicaux benzyle ou phényle, le noyau aromatique des groupes benzyles ou phényles pouvant être substitués par un ou plusieurs radicaux choisis parmi les radicaux alkyles en $C_1$-$C_4$, alcoxy en $C_1$-$C_4$, hydroxyalkyle en $C_1$-$C_4$, amino ou halogène. De préférence, ces substituants sont choisis parmi les radicaux alkyles ou hydroxyalkyles en $C_1$-$C_4$, linéaires ou ramifiés.

L'hétérocycle ou la partie hétérocyclique du groupement A est éventuellement substituée par au moins un radical choisi parmi les radicaux alkyle linéaires ou ramifiés en $C_1$-$C_6$, de préférence méthyle, éthyle ; les radicaux hydroxyle, amino et alcoxy en $C_1$-$C_4$, halogène. De préférence, les radicaux sont des radicaux alkyle en $C_1$-$C_4$, linéaire ou ramifié.

La partie aromatique du groupement A est éventuellement substituée par au moins un radical choisi parmi les radicaux trifluorométhyle ; alcoxy($C_1$-$C_6$)carbonyle ; nitro, hydroxy, alkyle en $C_1$-$C_4$, alcoxy en $C_1$-$C_4$, amino, halogène.

Le groupement B peut être éventuellement substitué par au moins un radical choisi parmi les radicaux alkyle linéaires ou ramifiés en $C_1$-$C_6$ ; les radicaux alcoxy linéaires ou ramifiés en $C_1$-$C_6$ ; les radicaux amino ; les radicaux amino substitués par un ou deux radicaux alkyle, identiques ou non, linéaires ou ramifiés en $C_1$-$C_6$ ; les radicaux phénylamino pour lesquels le groupement phényle peut être substitué par un ou plusieurs radicaux alkyle en $C_1$-$C_6$, alcoxy en $C_1$-$C_6$, halogène, amine éventuellement substitué par un ou plusieurs groupements alkyle en $C_1$-$C_6$ identiques ou non ; les groupements hydroxyle ; les atomes d'halogène ; les groupements nitro.

Dans le cas où B est un groupement anthraquinone rattaché par l'un des atomes de carbone de l'un des noyaux aromatiques à l'atome d'azote de la fonction hydrazone, celui-ci peut alors être substitué, de préférence sur l'autre noyau aromatique, par un groupement de formule A-C($R_3$)=N-N($R_1$)- avec A, $R_3$ et $R_1$ ayant la même signification que précédemment et étant choisis de telle sorte qu'ils soient respectivement identiques à ceux de l'autre partie de la molécule de formule (I).

Selon un mode de réalisation particulier de l'invention, dans le cas d'un groupement B de type anthraquinone, le radical $R_1$ représente un atome d'hydrogène.

Selon un autre mode de réalisation avantageux de l'invention, lorsque le groupement A est un indole et le groupement B est un groupement de type carbazole, alors le groupement A est substitué sur la partie aromatique du groupement, comme décrit précédemment.

L'anion ou le mélange d'anions cosmétiquement acceptables, X, est par exemple (sont) choisi(s) parmi les halogénures tels que chlorures, bromures, fluorures, iodures ; les hydroxyde ; les sulfates ; les hydrogénosulfates ; les alkyl($C_1$-$C_6$)sulfates ; les phosphates ; les carbonates ; les hydrogénocarbonates ; les perchlorates ; les acétates ; les tartrates ; les citrates, les oxalates ; les alkyl($C_1$-$C_6$)sulfonates tels que le méthylsulfonate ; les arylsulfonates substitués ou non par un radical alkyle en $C_1$-$C_4$ tels que par exemple un 4-toluylsulfonate ; les trichlorozincates.

Les colorants de l'invention sont des composés connus en eux-mêmes. Parmi ceux-ci, on peut citer les composés suivants, ainsi que leurs formes tautomères :

| | |
|---|---|
| 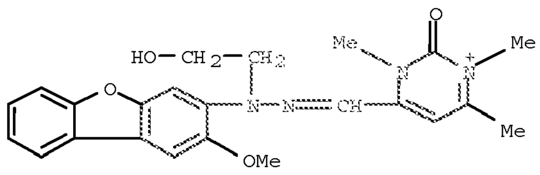 X | 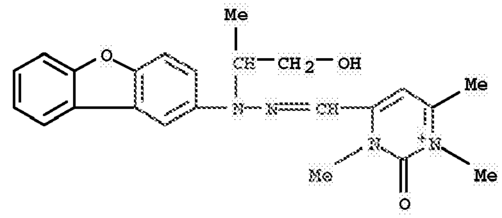 X |
| Sel de Pyrimidinium, 2,3-dihydro-4-[[(2-hydroxyethyl)(2-methoxy-3-dibenzofuranyl) hydrazono]methyl]-1,3,6-trimethyl-2-oxo | Sel de Pyrimidinium, 4-[[2-dibenzofuranyl(2-hydroxy-1-methylethyl)hydrazono] methyl]-2,3-dihydro-1,3,6-trimethyl-2-oxo- |
| 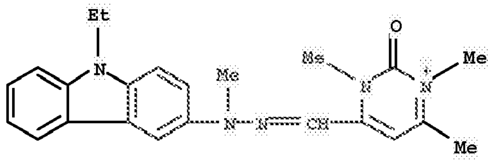 X | 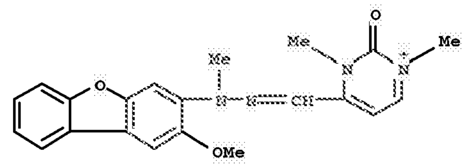 X |
| Sel de Pyrimidinium, 4-[[(9-ethyl-9H-carbazol-3-yl)methylhydrazono]methyl]-2,3-dihydro-1,3,6-trimethyl-2-oxo- (9Cl) | Sel de Pyrimidinium, 2,3-dihydro-4-[[(2-methoxy-3-dibenzofuranyl)methylhydrazono] methyl]-1,3-dimethyl-2-oxo- (9Cl) |
| 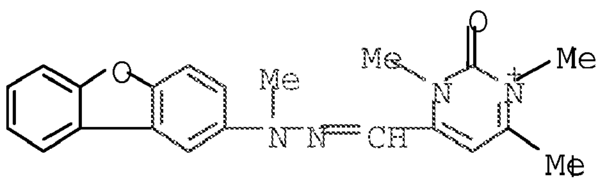 X | 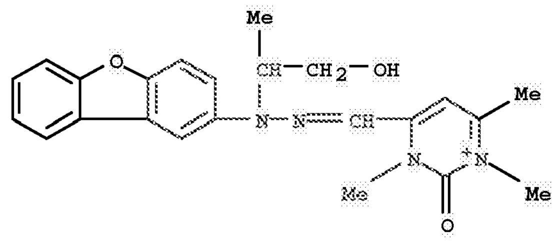 |
| | 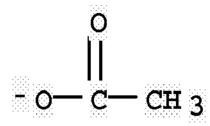 |
| Sel de Pyrimidinium, 4-[(2-dibenzofuranyl methylhydrazono)methyl]-2,3-dihydro-1,3,6-trimethyl-2-oxo- (9Cl) | Acétate de Pyrimidinium, 4-[[2-dibenzofuranyl(2-hydroxy-1-methylethyl) hydrazono] methyl]-2,3-dihydro-1,3,6-trimethyl-2-oxo- (9Cl) |

(suite)

| | |
|---|---|
| 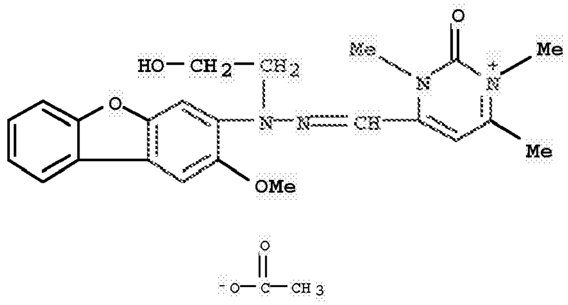 | 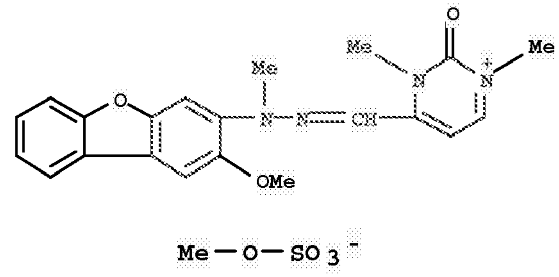 |
| Acétate de Pyrimidinium, 2,3-dihydro-4-[[(2-hydroxyethyl)(2-methoxy-3-dibenzofuranyl) hydrazono]methyl]-1,3,6-trimethyl-2-oxo (9Cl) | Methyl sulfate de Pyrimidinium, 2,3-dihydro-4-[[(2-methoxy-3-dibenzofuranyl)methyl hydrazono]methyl]-1,3-dimethyl-2-oxo (9Cl) |
| 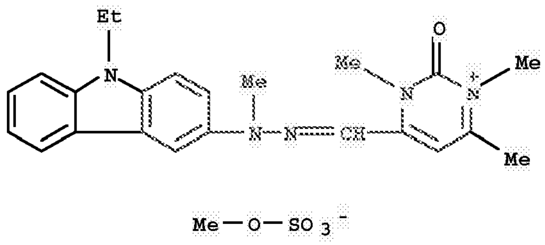 | 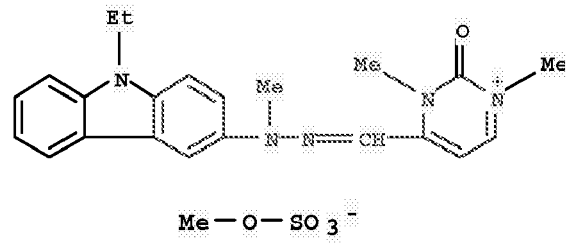 |
| methyl sulfate de Pyrimidinium, 4-[[(9-ethyl-9H-carbazol-3-yl)methylhydrazono] methyl]-2,3-dihydro-1,3,6-trimethyl-2-oxo (9Cl) | methyl sulfate de Pyrimidinium, 4-[[(9-ethyl-9H-carbazol-3-yl)methylhydrazono] methyl]-2,3-dihydro-1,3-dimethyl-2-oxo (9Cl) |
| 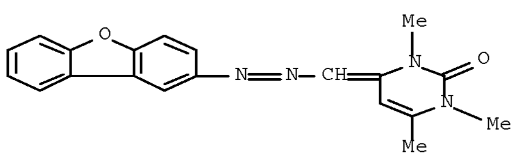 | 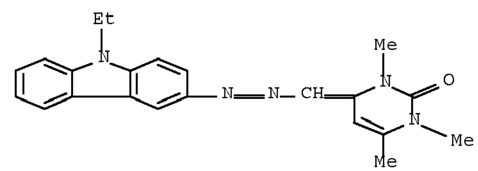 |
| 2(1 H)-Pyrimidinone, 4-[(2-dibenzofuranylazo)methylene]-3,4-dihydro-1,3,6-trimethyl | 2(1 H)-Pyrimidinone, 4-[[(9-ethyl-9H-carbazol-3-yl)azo]methylene]-3,4-dihydro-1,3,6-trimethyl |

(suite)

| | |
|---|---|
| 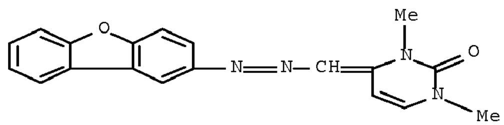 | 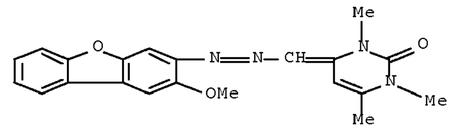 |
| 2(1 H)-Pyrimidinone, 4-[(2-dibenzofuranylazo)methylene]-3,4-dihydro-1,3-dimethyl | 2(1 H)-Pyrimidinone, 3,4-dihydro-4-[[(2-methoxy-3-dibenzofuranyl)azo]methylene]-1,3,6-trimethyl |
| 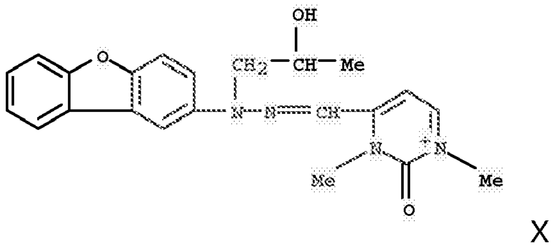 | 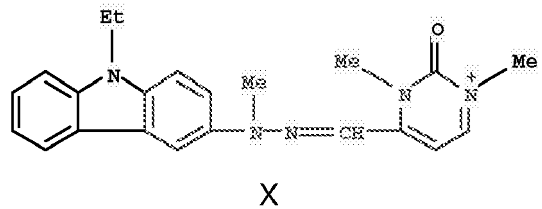 |
| Sel de Pyrimidinium, 4-[[2-dibenzofuranyl(2-hydroxypropyl)hydrazono]methyl]-2,3-dihydro-1,3-dimethyl-2-oxo- | Sel de Pyrimidinium, 4-[[(9-ethyl-9H-carbazol-3-yl)methylhydrazono]methyl]-2,3-dihydro-1,3-dimethyl-2-oxo (9Cl) |
| 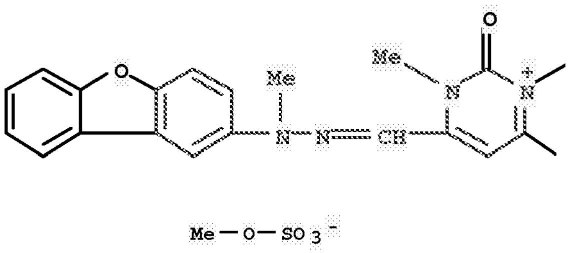 | 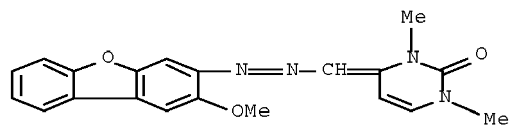 |
| Méthyl sulfate de Pyrimidinium, 4-[(2-dibenzofuranylmethylhydrazono)methyl]-2,3-dihydro-1,3,6-trimethyl-2-oxo (9Cl) | 2(1 H)-Pyrimidinone, 3,4-dihydro-4-[[(2-methoxy-3-dibenzofuranyl)azo]methylene]-1,3-dimethyl |
| 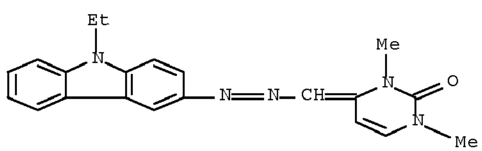 | |

(suite)

| | |
|---|---|
| 2(1H)-Pyrimidinone, 4-[[(9-ethyl-9H-carbazol-3-yl)azo]methylene]-3,4-dihydro-1,3-dimethyl | |
| 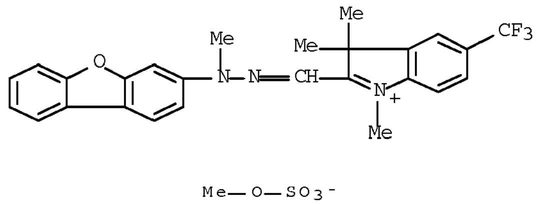 | 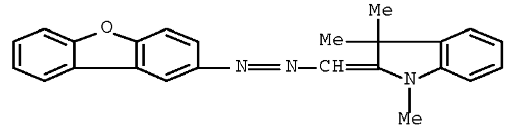 |
| methyl sulfate de 3H-Indolium, 2-[(2-dibenzofuranylmethylhydrazono)methyl]-5-trifluorométhyl)-1,3,3-trimethyl | 1H-Indole, 2-[(2-dibenzofuranylazo)methylene]-2,3-dihydro-1,3,3-trimethyl |
| 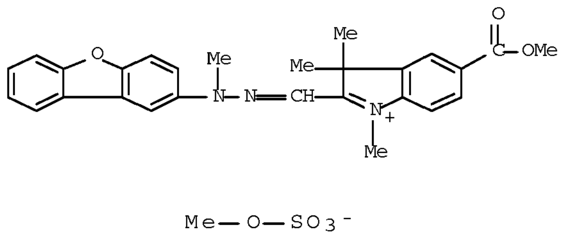 | 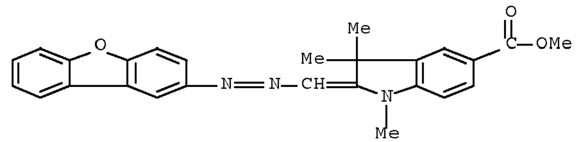 |
| methyl sulfate de 3H-Indolium, 2-[(2-dibenzofuranylmethylhydrazono)methyl]-5-methoxycarbonyl)-1,3,3-trimethyl-, | 1 H-Indole-5-carboxylic acid, 2-[(2-dibenzofuranylazo)methylene]-2,3-dihydro-1,3,3-trimethyl-, methyl ester |
| 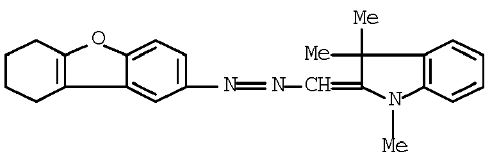 | 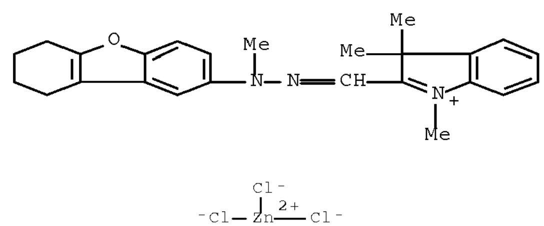 |
| 1 H-Indole, 2,3-dihydro-1,3,3-trimethyl-2-[[(6,7,8,9-tetrahydro-2-dibenzofuranyl) azo]methylene] | trichlorozincate(1-) de 3H-Indolium, 1,3,3-trimethyl-2-[[methyl(6,7,8,9-tetrahydro-2-dibenzofuranyl)hydrazono]methyl]-, |

(suite)

| | |
|---|---|
| 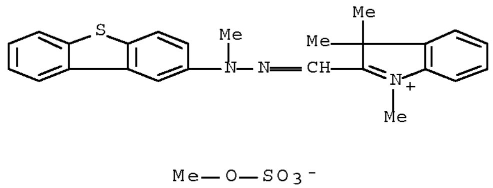 | 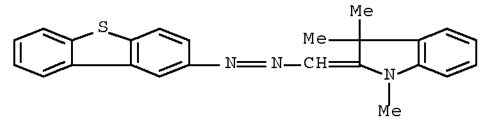 |
| methyl sulfate de 3H-Indolium, 2-[(2-dibenzothienylmethylhydrazono)methyl]-1,3,3-trimethyl | 1H-Indole, 2-[(2-dibenzothienylazo)methylene]-2,3-dihydro-1,3,3-trimethyl- |
| 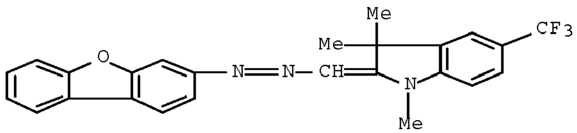 | 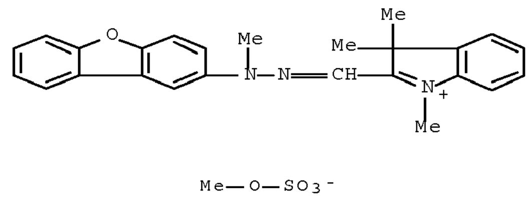 |
| 1 H-Indole, 2-[(3-dibenzofuranylazo)methylene]-2,3-dihydro-1,3,3-trimethyl-5-(trifluoromethyl)- | methyl sulfate de 3H-Indolium, 2-[(2-dibenzofuranylmethylhydrazono)methyl]-1,3,3-trimethyl |
| 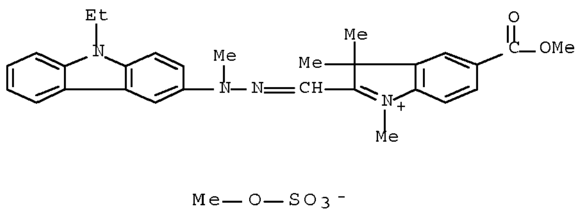 | 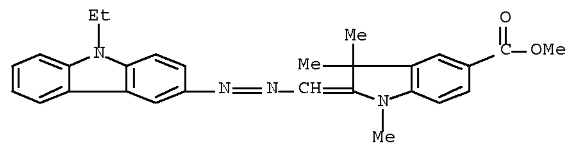 |
| methyl sulfate de 3H-Indolium, 2-[[(9-ethyl-9H-carbazol-3-yl)methylhydrazono] methyl]-5- (methoxycarbonyl)-1,3,3-trimethyl | 1 H-Indole-5-carboxylic acid, 2-[[(9-ethyl-9H-carbazol-3-yl)azo]methylene]-2,3-dihydro-1,3,3-trimethyl-, methyl ester |

(suite)

| | |
|---|---|
| 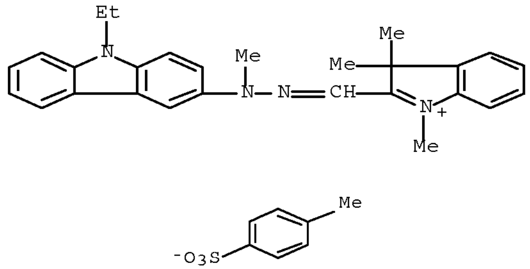 | 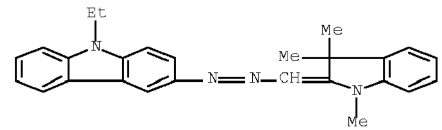 |
| 4-methylbenzenesulfonate de 3H-Indolium, 2-[[(9-ethyl-9H-carbazol-3-yl)methylhydrazono]methyl]-1,3,3-trimethyl | C9H-Carbazole, 3-[[(1,3-dihydro-1,3,3-trimethyl-2H-indol-2-ylidene)methyl]azo]-9-ethyl- |
| 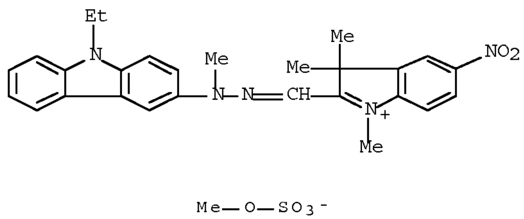 | 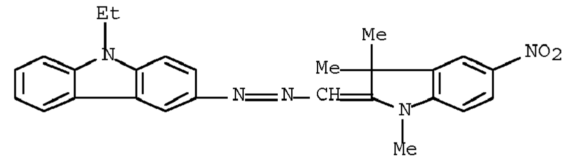 |
| Méthylesulfate de 3H-Indolium, 2-[[(9-ethyl-9H-carbazol-3-yl)methylhydrazono]methyl]-1,3,3-trimethyl-5-nitro | 9H-Carbazole, 3-[[(1,3-dihydro-1,3,3-trimethyl-5-nitro-2H-indol-2-ylidene)methyl]azo]-9-ethyl- |
| 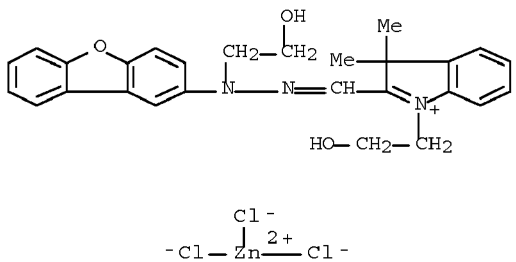 | 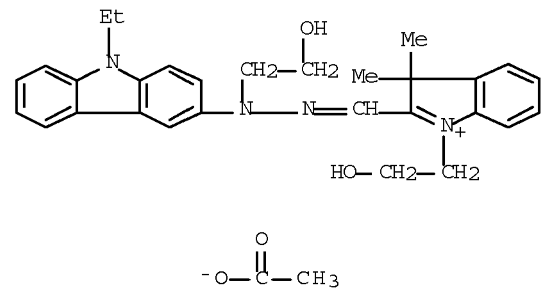 |
| trichlorozincate de 3H-Indolium, 2-[[2-dibenzofuranyl(2-hydroxyethyl)hydrazono]methyl]-1-(2-hydroxyethyl)-3,3-dimethyl | acetate de 3H-Indolium, 2-[[(9-ethyl-9H-carbazol-3-yl)(2-hydroxyethyl)hydrazono]methyl]-1-(2-hydroxyethyl)-3,3-dimethyl |

(suite)

| | |
|---|---|
| 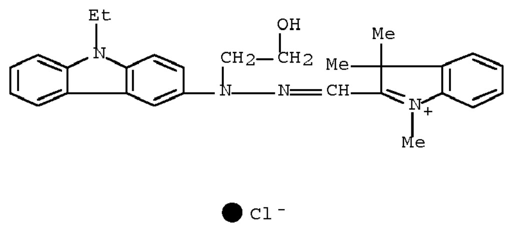 | |
| Chlorure de 3H-Indolium, 2-[[(9-ethyl-9H-carbazol-3-yl)(2-hydroxyethyl)hydrazono] methyl]-1,3,3-trimethyl | |
| 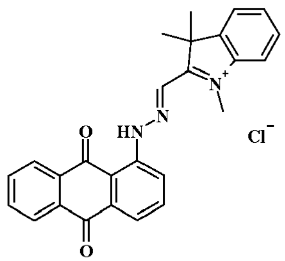 | 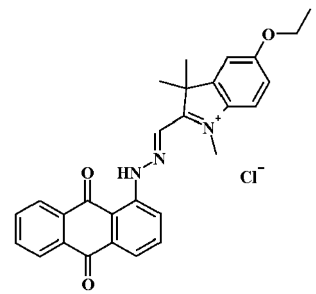 |
| Chlorure de 2-[(1-anthraquinonyl hydrazono)methyl]-1,3,3-trimethyl-3H-Indolium | Chlorure de 2-[(1-anthraquinonylhydrazono) methyl]-5-ethoxy-1,3,3-trimethyl-3H-Indolium |
| 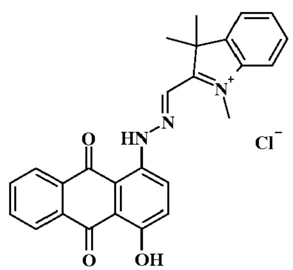 | 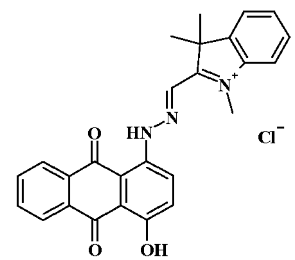 |
| Chlorure de 2-[(4-hydroxy-1-anthraquinonyl hydrazono)methyl]-1,3,3-trimethyl-3H-Indolium | Chlorure de 2-[(4-methoxy-1-anthraquinonyl hydrazono)methyl]-1,3,3-trimethyl- 3H-Indolium |

(suite)

| | |
|---|---|
| 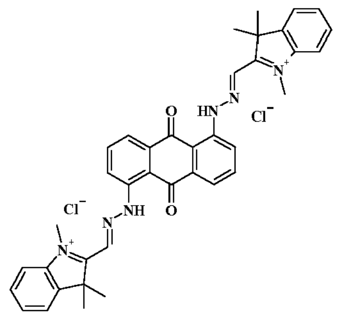 | 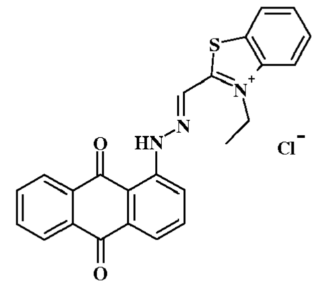 |
| Chlorure de 2,2'-[1,5-anthraquinonylenebis (iminonitrilomethylidyne)]bis[1,3,3-trimethyl-3H-Indolium] | Chlorure de 2-[(1-anthraquinonyl hydrazono)methyl]-3-ethyl-benzothiazolium |

A noter que la nature des contre-ions présents dans le tableau ci-dessus, lorsqu'elle est précisée, est simplement donnée à titre indicatif et que dans le cadre de l'invention, on envisage tous les colorants cités ci-dessus associés au contre-ion X tel que défini précédemment.

Les composés mis en oeuvre dans le cadre de l'invention peuvent notamment être obtenus à partir d'un aldéhyde ou d'une cétone et d'un dérivé d'hydrazine en deux étapes : condensation puis quaternisation.

Les composés mis en oeuvre dans l'invention peuvent être aussi obtenus à partir d'un méthylène activé et d'un dérivé de sel de diazonium en une étape.

Conformément à un mode de réalisation avantageux de l'invention, la concentration en colorant(s) direct(s) de formule (I) peut varier de 0,001% à 5% en poids par rapport au poids de la composition tinctoriale, et de préférence de 0,05% à 2% en poids par rapport au poids de la composition tinctoriale.

Comme indiqué auparavant, la composition tinctoriale selon l'invention comprend par ailleurs au moins un agent épaississant. Par agent épaississant, on entend au sens de la présente invention tout agent connu pour augmenter la viscosité lorsqu'il est placé dans un milieu aqueux. En particulier, de par leur présence, ces agents épaississants augmentent la viscosité d'un milieu aqueux d'au moins 50 cps à 25°C à un taux de cisaillement de 1 $s^{-1}$ s'ils sont présents à 1 % en poids de matière active.

Les agents épaississant sont notamment choisis parmi :

(i) les épaississants associatifs;
(ii) les homopolymères d'acide acrylique réticulés ;
(iii) les copolymères réticulés d'acide (méth)acrylique et d'acrylate d'alkyle($C_1$-$C_6$) ;
(iv) les homopolymères et copolymères non-ioniques contenant des monomères à insaturation éthylénique de type ester et/ou amide ;
(v) les homopolymères d'acrylate d'ammonium ou les copolymères d'acrylate d'ammonium et d'acrylamide ;
(vi) les polysaccharides ;
(vii) les alcools gras en $C_{12}$-$C_{30}$ ;
(viii) les épaississants minéraux ;

ou leurs mélanges.

Par l'expression "épaississant associatif", on entend un épaississant amphiphile comportant à la fois des motifs hydrophiles et des motifs hydrophobes en particulier comportant au moins une chaîne grasse en $C_8$-$C_{30}$, de préférence en $C_{10}$-$C_{30}$. De préférence, la chaîne grasse est une chaîne alkyle ou comprend une chaîne alkyle.

On peut utiliser comme épaississants associatifs les polymères associatifs choisis parmi :

(i) les polymères amphiphiles non ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse.

Les polymères amphiphiles non ioniques comportant au moins une chaîne grasse et au moins un motif hydrophile sont choisis de préférence parmi :

(1) les celluloses modifiées par des groupements comportant au moins une chaîne grasse. On peut citer à titre d'exemple :

- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$. De préférence, la chaîne grasse est une chaîne alkyle en $C_8$-$C_{22}$. A titre d'exemple de ce type de produit, on peut citer le NATROSOL PLUS GRADE 330 CS (alkyles en $C_{16}$) vendu par la société AQUALON, ou le BERMOCOLL EHM 100 vendu par la société BEROL NOBEL,
- celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

(2) les hydroxypropylguars modifiés par des groupements comportant au moins une chaîne grasse tel que le produit ESAFLOR HM 22 (chaîne alkyle en $C_{22}$) vendu par la société LAMBERTI, les produits MIRACARE XC95-3 (chaîne alkyle en $C_{14}$) et RE205-1 (chaîne alkyle en $C_{20}$) vendus par la société RHODIA CHIMIE.

(3) les uréthanes polyéthers comportant au moins une chaîne grasse telle que des groupes alkyle ou alcényle en $C_{10}$-$C_{30}$, comme les produits DAPRAL T 210 et DAPRAL T 212 vendus par la société AKZO ou les produits ACULYN 44 et ACULYN 46 commercialisés par la société ROHM&HAAS.

(4) les copolymères de vinyl pyrrolidone et de monomères hydrophobes à chaîne grasse. On peut citer à titre d'exemple :

- les produits ANTARON V216 ou GANEX V216 (copolymère vinylpyrrolidone / hexadécène) vendu par la société I.S.P.
- les produits ANTARON V220 ou GANEX V220 (copolymère vinylpyrrolidone / eicosène) vendu par la société I.S.P.

(5) Les copolymères de méthacrylates ou d'acrylates d'alkyles en $C_1$-$C_6$ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.

(6) Les copolymères de méthacrylates ou d'acrylates hydrophiles et de monomères hydrophobes comportant au moins une chaîne grasse, tels que par exemple le copolymère méthacrylate de polyéthylèneglycol/méthacrylate de lauryle.

Parmi les polymères amphiphiles anioniques susceptibles d'être mis en oeuvre et comportant au moins un motif hydrophile, et au moins un motif à chaîne grasse, on préfère ceux comportant au moins un motif éther d'allyle à chaîne grasse et au moins un motif hydrophile constitué par un monomère anionique insaturé éthylénique, plus particulièrement par un acide carboxylique vinylique et tout particulièrement par un acide acrylique, un acide méthacrylique ou leurs mélanges. Le motif éther d'allyle à chaîne grasse correspond plus particulièrement au monomère de formule (V) suivante :

$$CH_2 = C(R_1)\ CH_2\ O\ B_n\ R \qquad (V)$$

dans laquelle R1 désigne H ou $CH_3$, B désigne le radical éthylèneoxy, n est nul ou désigne un entier allant de 1 à 100, R désigne un radical hydrocarboné choisi parmi les radicaux alkyl, arylalkyl, aryl, alkylaryl, cycloalkyl, comprenant de 10 à 30 atomes de carbone, de préférence 10 à 24, et plus particulièrement encore de 12 à 18 atomes de carbone.

Un motif de formule (V) plus particulièrement préféré selon la présente invention est un motif dans lequel R1 désigne H, n est égal à 10, et R désigne un radical stéaryle ($C_{18}$).

Des polymères amphiphiles anioniques de ce type sont décrits et préparés, selon un procédé de polymérisation en émulsion, dans la demande de brevet EP-0216479.

Parmi ces polymères amphiphiles anioniques décrits ci dessus, on préfère particulièrement les polymères formés à partir de 20 à 60% en poids d'acide acrylique et/ou d'acide méthacrylique, de 5 à 60% en poids de (méth) acrylates d'alkyls inférieurs, de 2 à 50% en poids d'éther d'allyle à chaîne grasse de formule (V), et de 0 à 1% en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques peuvent être également choisis parmi ceux comportant au moins un motif hydrophile de type acide carboxylique insaturé oléfinique, et au moins un motif hydrophobe de type ester d'alkyl ($C_{10}$-$C_{30}$) d'acide carboxylique insaturé. Ils sont choisis de préférence parmi ceux dont le motif hydrophile de type acide carboxylique insaturé oléfinique correspond au monomère de formule (VI) suivante :

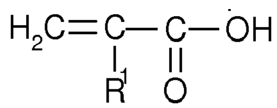 (IV) (VI)

formule dans laquelle, $R^1$ désigne H ou $CH_3$ ou $C_2H_5$, c'est-à-dire des motifs acide acrylique, acide méthacrylique ou acide éthacrylique et dont le motif hydrophobe de type ester d'alkyl ($C_{10}$-$C_{30}$) d'acide carboxylique insaturé correspond au monomère de formule (VII) suivante :

$H_2C=CR^1\text{-CO-OR}^2$ (VII)

formule dans laquelle, $R^1$ désigne H ou $CH_3$ ou $C_2H_5$ (c'est-à-dire des motifs acrylates, méthacrylates ou éthacrylates) et de préférence H ou $CH_3$, $R^2$ désignant un radical alkyle en $C_{10}$-$C_{30}$, et de préférence en $C_{12}$-$C_{22}$.

Des esters d'alkyle ($C_{10}$-$C_{30}$) d'acides carboxyliques insaturés comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques du type que ceux décrits ci dessus sont par exemple décrits et préparés, selon les brevets US-3 915 921 et 4 509 949.

Les polymères amphiphiles anioniques du même type que ceux décrits ci dessus utilisables dans le cadre de la présente invention peuvent aussi désigner des polymères formés à partir d'un mélange de monomères comprenant :

(i) essentiellement de l'acide acrylique, un ester de formule (VIII) suivante :

$H_2C=CR^1\text{-CO-OR}^2$ (VIII)

dans laquelle $R^1$ désigne H ou $CH_3$, $R^2$ désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou 98 à 96% en poids d'acide acrylique (motif hydrophile), 1 à 4% en poids d'acrylate d'alkyles en $C_{10}$-$C_{30}$ (motif hydrophobe), (ii) essentiellement de l'acide acrylique et du méthacrylate de lauryle tel que celui formé à partir de 66% en poids d'acide acrylique et 34% en poids de méthacrylate de lauryle.

Ces polymères peuvent être réticulés à l'aide d'agent réticulants. Cet réticulant sont des composés contenant un groupe

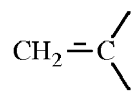

avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Comme polymères amphiphiles anioniques à chaînes grasses, on peut également citer le copolymère acide méthacrylique/acrylate de méthyle/diméthylméta-isopropénylbenzyl isocyanate d'alcool éthoxylé commercialisé sous la dénomination VISCOPHOBE DB 1000 par la société AMERCHOL.

Les polymères amphiphiles cationiques utilisés dans la présente invention sont choisis de préférence parmi les dérivés de cellulose quaternisée et les polyacrylates à groupements latéraux aminés.

Les dérivés de cellulose quaternisée sont, en particulier,

- les celluloses quaternisées modifiées par des groupements comportant au moins une chaîne grasse, tels que les groupes alkyle, arylalkyle, alkylaryle, et de préférence alkyle, comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle, et de préférence alkyle, comportant au moins 8 atomes de carbone, ou des mélanges de ceux-ci.

Les polyacrylates à groupements latéraux aminés, quaternisés ou non, possèdent par exemple des groupements hydrophobes du type stéareth 20 (alcool stéarylique polyoxyéthyléné (20)) ou alkyl(C10-C30)PEG-20 itaconate.

Les radicaux alkyles porté par les celluloses ou hydroxyéthylcelluloses quaternisées ci-dessus comportent de préférence de 8 à 30 atomes de carbone.

Les radicaux aryle désignent de préférence les groupements phényle, benzyle, naphtyle ou anthryle.

On peut indiquer comme exemples d'alkylhydroxyéthylcelluloses quaternisées à chaînes grasses en $C_8$-$C_{30}$, les

produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en $C_{18}$) commercialisés par la société CRODA;

Comme exemples de polyacrylates à chaînes latérales aminées, on peut citer les polymères 8781-124B ou 9492-103 ou STRUCTURE PLUS de la société NATIONAL STARCH.

A titre de polymères amphiphiles amphotères contenant au moins une chaîne grasse, on peut citer les copolymères de chlorure de méthacrylamidopropyl triméthylammonium/acide acrylique/méthacrylate d'alkyle en $C_{10}$-$C_{30}$, le radical alkyle étant de préférence un radical stéaryle.

(ii) Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.

(iii) Parmi les copolymères réticulés d'acide (méth)acrylique et d'acrylate d'alkyle en $C_1$-$C_6$, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active.

(iv) Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle / diméthacrylate d'éthylèneglycol); ACRYLOID B66 par la société RHOM & HAAS (copolymère méthacrylate de butyle / méthacrylate de méthyle); BPA 500 par la société KOBO (polyméthacrylate de méthyle).

(v) Parmi les homopolymères d'acrylate d'ammonium, on peut citer le produit vendu sous le nom MICROSAP PAS 5193 par la société HOECHST.

Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).

(vi) Les polysaccharides épaississants sont notamment choisis parmi les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénanes, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et les gommes de xanthane, et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980" et dans l'Industrial Gums - Polysaccharides and their Dérivatives, Edité par Roy L. WHISTLER, Second Edition, Edition Academic Press Inc.", le contenu de ces trois ouvrages étant totalement inclus dans la présente demande à titre de référence.

On utilisera de préférence parmi les polysaccharides, les amidons, les gommes de guar, les celluloses et leurs dérivés.

Les gommes de guar peuvent être modifiées ou non modifiées.

Les gommes de guar non modifiées sont par exemple les produits vendus sous la dénomination VIDOGUM GH 175 par la société UNIPECTINE, et sous les dénominations MEYPRO-GUAR 50 et JAGUAR C par la société MEYHALL.

Les gommes de guar non ioniques modifiées sont notamment modifiées par des groupements hydroxyalkyle en $C_1$-$C_6$.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le

nombre de fonctions hydroxyle libres présentes sur la gomme de guar, varie de préférence de 0,4 à 1,2.

De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHODIA CHIMIE (MEYHALL) ou sous la dénomination GALACTASOL 4H4FD2 par la société AQUALON.

Parmi les celluloses et leurs dérivés, on utilise notamment les celluloses modifiées par des groupements hydroxyalkyle en $C_1$-$C_6$, et tout particulièrement les hydroxyéthylcelluloses, les hydroxypropylcelluloses. On peut citer par exemple les produits vendus sous les dénominations KLUCEL EF, KLUCEL H, KLUCEL LHF, KLUCEL MF, KLUCEL G, par la société AQUALON.

Les alcools gras sont notamment choisis parmi les alcools monohydroxyxlés comprenant de 8 à 30 atomes de carbone, de préférence saturés, comme par exemple l'alcool myristylique, l'alcool cétylique, l'alcool stéarylique, l'alcool béhénylique.

Les épaississants minéraux peuvent aussi être utilisés et sont notamment choisis parmi les argiles.

De préférence, les agents épaississants de l'invention sont des polymères organiques.

Selon l'invention, le ou les agents épaississant peuvent représenter de 0,001 % à 20 % en poids, de préférence de 0,01 % à 10% en poids et plus particulièrement de 0,1 à 3% en poids par rapport au poids de la composition tinctoriale.

Lorsque la composition de l'invention contient un agent tensioactif, cet agent tensioactif peut être choisi parmi les agents tensioactifs anioniques, amphotères, non ioniques, cationiques ou leurs mélanges.

Les tensioactifs convenant à la mise en oeuvre de la présente invention sont notamment les suivants :

(i) <u>Tensioactif(s) anionique(s)</u> :

A titre d'exemple de tensioactifs anioniques utilisables, seuls ou mélanges, dans le cadre de la présente invention, on peut citer notamment les sels (en particulier sels alcalins ou alcalino-terreux, notamment de sodium, de magnésium ; les sels d'ammonium ; les sels d'amines ; les sels d'aminoalcools) des composés suivants : les alkylsulfates ; les alkyléthersulfates ; les alkylamidoéthersulfates ; les alkylarylpolyéthersulfates ; les monoglycérides sulfates ; les alkylsulfonates ; les alkylphosphates ; les alkylamidesulfonates ; les alkylarylsulfonates ; les $\alpha$-oléfinesulfonates ; les paraffine-sulfonates ; les alkylsulfosuccinates ; les alkyléthersulfosuccinates ; les alkylamidesulfosuccinates ; les alkylsulfosuccinamates ; les alkylsulfoacétates ; les alkylétherphosphates ; les acylsarcosinates ; les acyliséthionates et les N-acyltaurates.

Parmi les tensioactifs anioniques encore utilisables, on peut également citer les sels d'acides gras saturés ou insaturés, comprenant de 8 à 30 atomes de carbone et éventuellement hydroxylés, tels que les sels des acides oléique, ricinoléique, palmitique, stéarique, les acides d'huile de coprah ou d'huile de coprah hydrogénée ; les acyllactylates.

A noter que le ou les radicaux alkyle, acyle de tous ces différents composés comportent de préférence de 8 à 24 atomes de carbone. Les radicaux aryle désignent de préférence un groupement phényle ou benzyle.

On peut également utiliser des tensioactifs faiblement anioniques, comme les acides d'alkyl($C_6$-$C_{24}$) D-galactoside uroniques et leurs sels ainsi que les acides alkyl ($C_6$-$C_{24}$) éther carboxyliques polyoxyalkylénés, les acides alkyl ($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) <u>Tensioactif(s) non ionique(s)</u> :

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178).

Ils peuvent être notamment choisis parmi les alcools polyéthoxylés, polypropoxylés ou polyglycérolés ; les alpha-diols polyéthoxylés, polypropoxylés ou polyglycérolés ; les alkylphénols polyéthoxylés, polypropoxylés ou polyglycérolés ou les acides gras polyéthoxylés, polypropoxylés ou polyglycérolés ; ces composés présentent une chaîne grasse, de préférence alkyle, comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les copolymères d'oxyde d'éthylène et de propylène, les condensats d'oxyde d'éthylène et de propylène sur des alcools gras, notamment saturés ou insaturés en $C_8$-$C_{30}$, mono- ou poly-hydroxylés ; les amides gras notamment saturés ou non, en $C_8$-$C_{30}$ polyéthoxylés ayant de préférence de 2 à 30 moles d'oxyde d'éthylène, les amides gras notamment saturés ou insaturés en $C_8$-$C_{30}$, polyglycérolés comportant en moyenne 1 à 5 groupements glycérol et en particulier 1,5 à 4 ; les esters d'acides gras notamment saturés ou non, en $C_8$-$C_{30}$ du sorbitan, oxyéthylénés ayant de 2 à 30 moles d'oxyde d'éthylène ; les esters d'acides gras notamment saturés ou non, en $C_8$-$C_{30}$ du sucrose ;

les esters d'acides gras notamment saturés ou non, en $C_8$-$C_{30}$ du polyéthylèneglycol ; les alkyl($C_8$-$C_{30}$)polyglycosides ; les dérivés de N-alkyl($C_8$-$C_{30}$)glucamine ; les oxydes d'amines tels que les oxydes d'alkyl($C_{10}$-$C_{14}$) amines ou les oxydes de N-acyl($C_8$-$C_{30}$)aminopropylmorpholine.

On notera que les alkylpolyglycosides constituent des tensioactifs non ioniques rentrant particulièrement bien dans le cadre de la présente invention.

(iii) Tensioactif(s) amphotère(s):

Les agents tensioactifs amphotères peuvent être notamment choisis parmi des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl($C_8$-$C_{20}$) bétaïnes, les sulfobétaïnes, les alkyl($C_8$-$C_{20}$) amidoalkyl($C_1$-$C_6$)bétaïnes ou les alkyl($C_8$-$C_{20}$)amidoalkyl($C_1$-$C_6$)sulfobétaïnes.

Parmi les dérivés d'amines, on peut citer les produits commercialisés sous les dénominations MIRANOL, tels que décrits dans les brevets US-2 528 378 et US-2 781 354 et de structures :

$$R_2\text{-}CONHCH_2CH_2\text{-}N(R_3)(R_4)(CH_2COO\text{-}) \qquad (2)$$

dans laquelle $R_2$ désigne un radical alkyle linéaire ou ramifié en $C_5$-$C_{20}$ provenant d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-}CONHCH_2CH_2\text{-}N(B)(C) \qquad (3)$$

dans laquelle B représente $-CH_2CH_2OX'$ C représente $-(CH_2)_z$ -Y', avec z = 1 ou 2, X' désigne le groupement $-CH_2CH_2$-COOH ou un atome d'hydrogène, Y' désigne -COOH ou le radical $-CH_2$ - CHOH - $SO_3H$, $R_5$ désigne un radical alkyle linéaire ou ramifié, saturé ou non, en $C_5$-$C_{20}$ provenant d'un acide $R_5$ -COOH notamment présent dans l'huile de coprah ou dans l'huile de lin hydrolysée.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHODIA CHIMIE.

(iv) Tensioactif(s) cationique(s) :

Les tensioactifs cationiques peuvent être choisis parmi :

A) les sels d'ammonium quaternaires de la formule générale (XII) suivante :

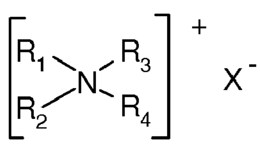

(XII)

dans laquelle :

X- est un anion choisi dans le groupe des halogénures (chlorure, bromure ou iodure) ou alkyl($C_2$-$C_6$)sulfates plus particulièrement méthylsulfate, des phosphates, des alkyl-ou-alkylarylsulfonates, des anions dérivés d'acide organique tel que l'acétate ou le lactate ; et

i) les radicaux $R_1$ à $R_3$, identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les

halogènes. Les radicaux aliphatiques sont par exemple choisis parmi les radicaux alkyle, alcoxy, alkylamide, $R_4$ désigne un radical alkyle, linéaire ou ramifié, comportant de 16 à 30 atomes de carbone.
De préférence le tensioactif cationique est un sel (par exemple chlorure) de béhényl triméthyl ammonium, ou
ii) les radicaux $R_1$ et $R_2$, identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyles, alcoxy, alkylamide et hydroxyalkyle, comportant environ de 1 à 4 atomes de carbone ;
$R_3$ et $R_4$, identiques ou différents, désignent un radical alkyle, linéaire ou ramifié, comportant de 12 à 30 atomes de carbone, ledit radical comprenant au moins une fonction ester ou amide ;
$R_3$ et $R_4$ sont notamment choisis parmi les radicaux alkyl($C_{12}$-$C_{22}$)amido alkyle($C_2$-$C_6$), alkyl($C_{12}$-$C_{22}$) acétate.

De préférence le tensioactif cationique est un sel (par exemple chlorure) de stéaramidopropyl diméthyl (myristylacétate) ammonium.
B) les sels d'ammonium quaternaire de l'imidazolinium, comme par exemple celui de formule (XIII) suivante :

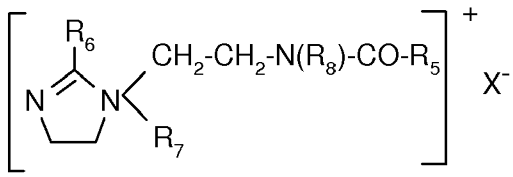 (XIII)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$, $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) les sels de diammonium quaternaire de formule (XIV) :

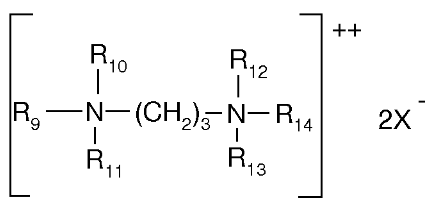 (XIV)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) les sels d'ammonium quaternaire contenant au moins une fonction ester de formule (XV) suivante :

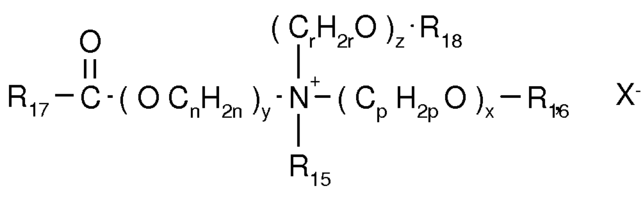 (XV)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical $R_{19}$-CO-
  - les radicaux $R_{20}$ hydrocarbonés en $C_1$-$C_{22}$ linéaires ou ramifiés, saturés ou insaturés,
  - l'atome d'hydrogène,
- $R_{18}$ est choisi parmi :
  - le radical $R_{21}$-CO-
  - les radicaux $R_{22}$ hydrocarbonés en $C_1$-$C_6$ linéaires ou ramifiés, saturés ou insaturés,
  - l'atome d'hydrogène,
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (XV) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :
  - le radical $R_{19}$-CO-
  - les radicaux méthyle, éthyle ou hydrocarbonés en $C_{14}$-$C_{22}$
  - l'atome d'hydrogène ;
- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :
  - le radical $R_{21}$-CO-
  - l'atome d'hydrogène.

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaires, on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

On utilise de préférence comme agent tensioactif anionique, les alkyl($C_{12}$-$C_{14}$) sulfates de sodium, de triéthanolamine ou d'ammonium ; les alkyl ($C_{12}$-$C_{14}$)éthersulfates de sodium, de triéthanolamine ou d'ammonium oxyéthylénés à 2,2 moles d'oxyde d'éthylène ; le cocoyl iséthionate de sodium et l'alphaoléfine($C_{14}$-$C_{16}$) sulfonate de sodium et leurs mélanges avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL® C2M CONC" en solution aqueuse à 38 % de matière active ou sous la dénomination MIRANOL® C32;

- soit un agent tensioactif amphotère tel que les alkylbétaïnes en particulier la cocobétaïne commercialisée sous la dénomination "DEHYTON® AB 30" en solution aqueuse à 32 % de MA par la société COGNIS ou tel que les alkyl ($C_8$-$C_{20}$) amidoalkyl ($C_1$-$C_6$) bétaïnes en particulier la TEGOBETAINE® F 50 commercialisée par la société GOLDSCHMIDT.

Avantageusement, la teneur en agent tensioactif varie entre 0,1% et 60% en poids, de préférence entre 3% et 40% en poids et encore plus préférentiellement entre 5% et 30% en poids par rapport au poids de la composition.

La composition tinctoriale selon l'invention peut éventuellement comprendre au moins un colorant direct additionnel différents des composés de formule (I). Celui-ci peut être choisi parmi les espèces cationiques ou non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Parmi les colorants directs azoïques utilisables selon l'invention on peut citer les colorants azoïques cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorant(s) direct(s) additionnels dans la composition tinctoriale varie en général de 0,001 à 20% en poids par rapport au poids de la composition, et de préférence de 0,005 à 10% en poids par rapport au poids de la composition.

La composition tinctoriale mise en oeuvre peut de plus comprendre une ou plusieurs bases d'oxydation.

Ces bases d'oxydation peuvent être choisies parmi les bases d'oxydation classiquement utilisées en teinture d'oxydation, par exemple les paraphénylènediamines, les bis-phénylalkylènediamines, les para-aminophénols, les orthoaminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques et les dérivés de type pyrazolo[1,2a]pyrazol-1-one.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés de type pyrazolo[1,2a]pyrazol-1-one, on peut citer les composés comme le 2,3-diamino-6,7-dihydro,1H-5H-pyrazolo[1,2a]pyrazol-1-one.

La ou les bases d'oxydation sont en général présentes en quantité comprise entre 0,001 à 10 % en poids par rapport au poids de la composition tinctoriale, et plus préférentiellement de 0,005 à 6 % en poids par rapport au poids de la composition tinctoriale.

La composition tinctoriale mise en oeuvre peut aussi comprendre un ou plusieurs coupleurs conventionnellement utilisés pour la teinture de fibres kératiniques humaines.

Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans ladite composition, le ou les coupleurs, s'ils sont présents, représentent en général une quantité comprise entre 0,001 et 10 % en poids par rapport au poids de la composition tinctoriale et plus préférentiellement de 0,005 à 6 % en poids par rapport au poids de la composition tinctoriale.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale mise en oeuvre comprend un milieu approprié pour la teinture, appelé aussi support de teinture. Il est généralement constitué par de l'eau ou par un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers

monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en $C_1$-$C_4$, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition tinctoriale.

La composition tinctoriale peut également renfermer divers adjuvants utilisés classiquement dans les compositions pour la teinture des cheveux, tels que des polymères anioniques, cationiques, non ioniques, amphotères, zwitterioniques ou leurs mélanges différents des polymères épaississants mentionnés auparavant, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition tinctoriale.

Le pH de la composition tinctoriale est généralement compris entre 3 et 12 environ, et de préférence entre 5 et 11 environ.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante :

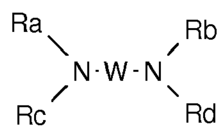

dans laquelle W est un reste propylène éventuellement substitué par un groupement hydroxyle ou un radical alkyle en $C_1$-$C_4$ $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques humaines.

La composition peut en outre comprendre au moins un agent oxydant.

Habituellement, ladite composition prête à l'emploi est obtenue en mélangeant la composition tinctoriale exempte d'agent oxydant avec une composition oxydante.

On peut aussi appliquer simultanément ou successivement une composition comprenant au moins un composé de formule (I) exempte d'agent oxydant, et une composition comprenant au moins un agent oxydant.

L'agent oxydant peut être n'importe quel agent de ce type utilisé de façon classique dans le domaine.

Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases.

L'utilisation du peroxyde d'hydrogène est particulièrement préférée.

La teneur en agent oxydant est en général comprise entre 1 et 40 % en poids, par rapport au poids de la composition prête à l'emploi de préférence entre 1 et 20 % en poids par rapport au poids de la composition prête à l'emploi.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques humaines.

Le procédé de l'invention peut être mis en oeuvre sur des fibres sèches ou humides.

Selon une première variante, l'invention concerne un procédé de coloration directe qui comprend l'application d'une composition tinctoriale contenant un colorant de formule (I) telle que définie précédemment sur les fibres kératiniques humaines, en l'absence d'agent oxydant, de base d'oxydation et de coupleur. Après un temps de pose, les fibres sont de préférence rincées laissant apparaître des fibres colorées.

Selon une deuxième variante, l'application sur les fibres de la composition tinctoriale contenant le colorant de

formule (I) peut également être mise en oeuvre en présence d'agent oxydant, mais en l'absence de base d'oxydation ou de coupleur, ce qui provoque la décoloration de la fibre (teinture directe éclaircissante). Comme indiqué auparavant, cet agent oxydant peut être ajouté à la composition contenant le colorant direct de formule (I) juste avant l'application ou directement sur la fibre.

L'invention a aussi pour objet un procédé de teinture d'oxydation dans lequel on l'applique sur les fibres kératiniques humaines, sèches ou humides, une composition tinctoriale comprenant un colorant de formule (I), au moins une base d'oxydation et optionnellement au moins un coupleur, en présence d'un agent oxydant.

Dans le cas de la teinture d'oxydation ou de la teinture directe éclaircissante, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration.

Le mélange obtenu est ensuite appliqué sur les fibres.

Quelle que soit la variante mise en oeuvre (avec ou sans agent oxydant), le temps de pose est généralement de l'ordre de 3 à 50 minutes, de préférence 5 à 30 minutes.

Le procédé peut comprendre des étapes supplémentaires tels qu'un étape de rinçage des fibres, une étape de lavage au shampooing, une étape de séchage, etc.

Un autre objet de l'invention est un dispositif à plusieurs compartiments, ou "kit", pour la teinture des fibres kératiniques humaines, et plus particulièrement des cheveux, dans lequel au moins un premier compartiment renferme la composition tinctoriale de l'invention exempte d'agent oxydant et au moins un deuxième compartiment renferme la composition oxydante.

Ce dispositif peut être équipé d'un moyen permettant de délivrer sur les cheveux le mélange souhaité, tel que les dispositifs décrits dans le brevet FR-2 586 913 au nom de la demanderesse.

A titre d'exemples de composition de l'invention, on peut préparer les compositions suivantes pour teindre des cheveux dans des nuances rouges.

Colorant (I) ou (II) 0.5g
Hydroxyéthylcellulose(NATROSOL 250 HHR d'AQUALON)) 1,0 g
Ethanol 5g
2-amino 2 méthyl 1 propanol qs pH 8
Eau qsp 100g

| |
|---|
| 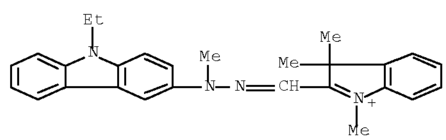 |
| 4-methylbenzenesulfonate de 3H-Indolium, 2-[[(9-ethyl-9H-carbazol-3-yl)methylhydrazono]methyl]-1,3,3-trimethyl-methyl sulfate (I) |
| 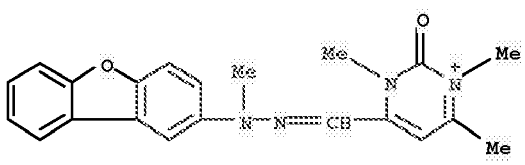 X |
| Sel (exemple méthyl sulfate) de Pyrimidinium, 4-[(2-dibenzofuranyl-methylhydrazono)methyl]-2,3-dihydro-1,3,6-trimethyl-2-oxo- (9Cl) (II) |

## Revendications

1. Composition tinctoriale, comprenant dans un milieu approprié pour la coloration des fibres kératiniques humaines :

(a) au moins un colorant hydrazonique cationique direct de formule (I), ou ses formes tautomères :

$$[A\text{-}C(R_3)\text{=}N\text{-}N(R_1)\text{-}B]^+ X$$

dans laquelle :

$R_1$ et $R_3$ indépendamment l'un de l'autre, représentent un atome d'hydrogène ; un radical alkyle linéaire ou ramifié en $C_1$-$C_{10}$ éventuellement porteur d'un groupement hydroxyle ; et de préférence représentent indépendamment l'un de l'autre un atome d'hydrogène ; un radical alkyle linéaire ou ramifié en $C_1$-$C_{10}$ ;
A représente un hétérocycle cationique condensé ou non, éventuellement substitué, choisi parmi les cycles pyrimidinone, indole, benzothiazole ; le cycle A étant relié au carbone de C(R3) par un atome de carbone ;
B représente un tricycle saturé ou insaturé, éventuellement substitué, choisi parmi les dibenzofuranes, les carbazoles, les anthraquinones ou les dibenzothiényles ; le tricycle, saturé ou insaturé, étant relié à l'atome d'azote de la fonction hydrazone par l'intermédiaire d'un atome de carbone de l'un des trois cycles ;
X représente un anion ou un mélanges d'anions cosmétiquement acceptables ;

(b) au moins un agent cosmétique choisi parmi les polymères épaississants et les tensio-actifs.

**2.** Composition selon la revendication 1, **caractérisée en ce que** le groupement A de la formule (1) est tel que l'une des formes tautomères présente un atome d'azote quaternisé engagé dans l'hétérocycle ; ledit atome d'azote portant un substituant choisi parmi les radicaux alkyle linéaires ou ramifiés en $C_1$-$C_{10}$ ; les radicaux hydroxyalkyle linéaires ou ramifiés en $C_1$-$C_{10}$ ; les radicaux benzyle et phényle, éventuellement substitué sur le cycle aromatique, le noyau aromatique des groupes benzyles ou phényles pouvant être substitués par un ou plusieurs radicaux choisis parmi les radicaux alkyles en $C_1$-$C_4$, alcoxy en $C_1$-$C_4$, hydroxyalkyle en $C_1$-$C_4$, amino ou halogène.

**3.** Composition selon l'une quelconque des revendications précédentes, **caractérisée en ce que** le groupement A de la formule (I) est tel que :

* l'hétérocycle ou la partie hétérocyclique du groupement A est éventuellement substituée par au moins un radical alkyle choisi parmi les radicaux alkyle linéaires ou ramifiés en $C_1$-$C_6$, les radicaux hydroxyle, amino et alcoxy en $C_1$-$C_4$, halogène,de préférence, les radicaux alkyle en $C_1$-$C_4$, linéaire ou ramifié et que
* la partie aromatique du groupement A est éventuellement substituée par au moins un radical choisi parmi les radicaux trifluorométhyle ; alcoxy($C_1$-$C_6$)carbonyle ; nitro, hydroxy, alkyle en $C_1$-$C_4$, alcoxy en $C_1$-$C_4$, amino, halogène.

**4.** Composition selon l'une quelconque des revendications précédentes, **caractérisée en ce que** le groupement B est éventuellement substitué par au moins un radical choisi parmi les radicaux alkyle linéaires ou ramifiés en $C_1$-$C_6$ ; les radicaux alcoxy linéaires ou ramifiés en $C_1$-$C_6$ ; les radicaux amino ; les radicaux amino substitués par un ou deux radicaux alkyle, identiques ou non, linéaires ou ramifiés en $C_1$-$C_6$ ; les radicaux phénylamino pour lesquels le groupement phényle peut être substitué ; les groupements hydroxyle ; les atomes d'halogène ; les groupements nitro.

**5.** Composition selon l'une quelconque des revendications précédentes, **caractérisée en ce que** si le groupement B est un groupement anthraquinone, alors ce groupement peut être substitué, de préférence sur l'autre noyau aromatique, par un groupement de formule

$$A\text{-}C(R_3)\text{=}N\text{-}N(R_1)\text{-}$$

avec A, $R_3$ et $R_1$ ayant la même signification que précédemment et étant choisis de telle sorte qu'ils soient respectivement identiques à ceux de l'autre partie de la molécule de formule (I).

**6.** Composition selon l'une quelconque des revendications précédentes, **caractérisée en ce que** si le groupement B est un groupement anthraquinone, le radical $R_1$ représente un atome d'hydrogène.

**7.** Composition selon l'une quelconque des revendications précédentes, **caractérisée en ce que** l'anion ou le mélange d'anions cosmétiquement acceptables, X, est (sont) choisi(s) parmi les halogénures ; les hydroxyde ; les sulfates ; les hydrogénosulfates ; les alkyl($C_1$-$C_6$)sulfates ; les phosphates ; les carbonates ; les hydrogénocarbonates ; les perchlorates ; les acétates ; les tartrates ; les citrates, les oxalates ; les alkyl($C_1$-$C_6$)sulfonates ; les arylsulfonates substitués ou non par un radical alkyle en $C_1$-$C_4$ les trichlorozincates.

**8.** Composition selon l'une quelconque des revendications précédentes, **caractérisée en ce que** la concentration en colorant(s) direct(s) de formule (I) varie de 0,001% à 5% en poids par rapport au poids total de la composition tinctoriale, et de préférence de 0,05% à 2% en poids par rapport au poids total de la composition tinctoriale.

**9.** Composition selon l'une quelconque des revendications précédentes, **caractérisée en ce que** l'agent épaississant est choisi parmi :

(i) les épaississants associatifs;
(ii) les homopolymères d'acide acrylique réticulés ;
(iii) les copolymères réticulés d'acide (méth)acrylique et d'acrylate d'alkyle($C_1$-$C_6$) ;
(iv) les homopolymères et copolymères non-ioniques contenant des monomères à insaturation éthylénique de type ester et/ou amide ;
(v) les homopolymères d'acrylate d'ammonium ou les copolymères d'acrylate d'ammonium et d'acrylamide ;
(vi) les polysaccharides ;
(vii) les alcools gras en $C_{12}$-$C_{30}$ ;
(viii) les épaississants minéraux,

ou leurs mélanges.

**10.** Composition selon la revendication 9 dans laquelle l'épaississant est un épaississant organique polymère.

**11.** Composition selon l'une quelconque des revendications précédentes, **caractérisée en ce que** le ou les agents épaississant représentent de 0,001 % à 20 % en poids, de préférence de 0,01 % à 10% en poids et plus particulièrement de 0,1 à 3% en poids par rapport au poids de la composition tinctoriale.

**12.** Composition selon l'une quelconque des revendications précédentes dans laquelle les agents tensio-actifs sont choisis parmi les tensio-actifs anioniques, amphotères, cationiques ou leurs mélanges

**13.** Composition selon la revendication 12 dans laquelle la quantité de tensio-actifs est comprise entre 0,1 et 60 %, de préférence entre 5 et 30 %.

**14.** Composition selon l'une quelconque des revendications précédentes, **caractérisée en ce que** la composition comprend au moins un colorant direct additionnel différent du colorant de formule (I) choisi parmi les colorants nitrés, azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

**15.** Composition selon l'une quelconque des revendications précédentes, **caractérisée en ce que** la composition comprend au moins une base d'oxydation, notamment choisie parmi les o-phénylènediamines, les p-phénylènediamines, les bases doubles, les o-aminophénols, les p-aminophénols, les bases hétérocycliques, leurs sels d'addition avec un acide, ainsi que leurs mélanges, et éventuellement un coupleur choisi parmi les m-aminophénols, les m-phénylènediamines, les m-diphénols, les naphtols, les coupleurs hétérocycliques, leurs sels d'addition avec un acide, ainsi que leurs mélanges.

**16.** Composition selon l'une quelconque des revendications précédentes, **caractérisée en ce que** la composition comprend au moins un agent oxydant.

**17.** Procédé de coloration de fibres kératiniques humaines dans lequel on applique sur les fibres, une composition selon l'une quelconque des revendications précédentes.

**18.** Dispositif à plusieurs compartiments, dans lequel au moins un premier compartiment renferme la composition tinctoriale selon l'une quelconque des revendications 1 à 13 et au moins un deuxième compartiment renferme la composition oxydante.

**19.** Utilisation des colorants de formule (I) tels que définis à l'une quelconque des revendications précédentes pour la teinture des fibres kératiniques.

Office européen des brevets

# RAPPORT DE RECHERCHE EUROPEENNE

Numéro de la demande
EP 07 10 4860

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 0 970 687 A1 (OREAL [FR]) 12 janvier 2000 (2000-01-12) * revendications 1,2,6,7,9-31 * ----- | 1-19 | INV. A61K8/49 A61Q5/06 |
| Y | EP 0 970 685 A1 (OREAL [FR]) 12 janvier 2000 (2000-01-12) * revendications 1,5,6,9-28 * ----- | 1-19 | |
| Y | EP 0 970 684 A1 (OREAL [FR]) 12 janvier 2000 (2000-01-12) * revendications 1,5,6,9-50 * ----- | 1-19 | |
| Y | US 3 840 518 A (SCHMITT E ET AL) 8 octobre 1974 (1974-10-08) * revendications 1-16 * * colonne 7, ligne 3 - ligne 39 * ----- | 1-19 | |
| Y | US 3 860 583 A (SCHMITT ERNST) 14 janvier 1975 (1975-01-14) * revendications 1-8 * * colonne 18, ligne 35 - ligne 56 * ----- | 1-19 | DOMAINES TECHNIQUES RECHERCHES (IPC) |
| Y | US 3 812 107 A (BOEHMKE G ET AL) 21 mai 1974 (1974-05-21) * revendications 1-13 * * colonne 14, ligne 58 - colonne 15, ligne 18 * ----- | 1-19 | A61K A61Q |
| Y | GB 769 163 A (CIBA LTD) 27 février 1957 (1957-02-27) * le document en entier * ----- | 1-19 | |

Le présent rapport a été établi pour toutes les revendications

| Lieu de la recherche | Date d'achèvement de la recherche | Examinateur |
|---|---|---|
| Berlin | 25 juin 2007 | Siatou, Evangelia |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

**ANNEXE AU RAPPORT DE RECHERCHE EUROPEENNE RELATIF A LA DEMANDE DE BREVET EUROPEEN NO.** EP 07 10 4860

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-06-2007

| Document brevet cité au rapport de recherche | Date de publication | Membre(s) de la famille de brevet(s) | Date de publication |
|---|---|---|---|
| EP 0970687 A1 | 12-01-2000 | AT 297182 T | 15-06-2005 |
| | | AU 722556 B2 | 03-08-2000 |
| | | AU 3680699 A | 03-02-2000 |
| | | BR 9903124 A | 30-05-2000 |
| | | CA 2277345 A1 | 09-01-2000 |
| | | CN 1246330 A | 08-03-2000 |
| | | DE 69925675 D1 | 14-07-2005 |
| | | DE 69925675 T2 | 30-03-2006 |
| | | DK 970687 T3 | 11-07-2005 |
| | | ES 2244159 T3 | 01-12-2005 |
| | | FR 2780881 A1 | 14-01-2000 |
| | | HU 9902334 A2 | 28-03-2000 |
| | | JP 2000063246 A | 29-02-2000 |
| | | KR 20000011486 A | 25-02-2000 |
| | | PT 970687 T | 31-10-2005 |
| | | RU 2185142 C2 | 20-07-2002 |
| | | US 2005235433 A1 | 27-10-2005 |
| | | ZA 9904283 A | 10-01-2000 |
| EP 0970685 A1 | 12-01-2000 | AT 296608 T | 15-06-2005 |
| | | AU 723806 B2 | 07-09-2000 |
| | | AU 3677799 A | 03-02-2000 |
| | | BR 9903081 A | 09-05-2000 |
| | | CA 2277347 A1 | 09-01-2000 |
| | | CN 1246331 A | 08-03-2000 |
| | | DE 69925539 D1 | 07-07-2005 |
| | | DE 69925539 T2 | 11-05-2006 |
| | | ES 2244158 T3 | 01-12-2005 |
| | | FR 2780882 A1 | 14-01-2000 |
| | | HU 9902331 A2 | 28-03-2000 |
| | | JP 2000063248 A | 29-02-2000 |
| | | KR 20000011515 A | 25-02-2000 |
| | | RU 2179436 C2 | 20-02-2002 |
| | | ZA 9904142 A | 23-12-1999 |
| EP 0970684 A1 | 12-01-2000 | AT 297181 T | 15-06-2005 |
| | | AU 719497 B2 | 11-05-2000 |
| | | AU 3796699 A | 09-03-2000 |
| | | BR 9903212 A | 30-05-2000 |
| | | CA 2277353 A1 | 09-01-2000 |
| | | CN 1248434 A | 29-03-2000 |
| | | DE 69925674 D1 | 14-07-2005 |
| | | DE 69925674 T2 | 30-03-2006 |
| | | ES 2244157 T3 | 01-12-2005 |
| | | FR 2780883 A1 | 14-01-2000 |
| | | HU 9902330 A2 | 28-03-2000 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 07 10 4860

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-06-2007

| Document brevet cité au rapport de recherche | Date de publication | Membre(s) de la famille de brevet(s) | Date de publication |
|---|---|---|---|
| EP 0970684 A1 | | JP 2000086472 A | 28-03-2000 |
| | | JP 2004339236 A | 02-12-2004 |
| | | KR 20000011517 A | 25-02-2000 |
| | | PT 970684 T | 30-09-2005 |
| | | RU 2180833 C2 | 27-03-2002 |
| | | ZA 9904102 A | 25-01-2000 |
| US 3840518 A | 08-10-1974 | BE 771028 A1 | 16-12-1971 |
| | | CH 563493 A | 30-06-1975 |
| | | CH 1164971 D | 13-12-1974 |
| | | DE 2039492 A1 | 10-02-1972 |
| | | FR 2102139 A5 | 07-04-1972 |
| | | GB 1330376 A | 19-09-1973 |
| | | JP 54006571 B | 29-03-1979 |
| | | NL 7110765 A | 10-02-1972 |
| US 3860583 A | 14-01-1975 | BE 776459 A1 | 09-06-1972 |
| | | CH 606286 A5 | 31-10-1978 |
| | | CH 606283 A5 | 31-10-1978 |
| | | DE 2060614 A1 | 15-06-1972 |
| | | FR 2123267 A1 | 08-09-1972 |
| | | GB 1362066 A | 30-07-1974 |
| | | IT 951661 B | 10-07-1973 |
| | | JP 55029076 B | 31-07-1980 |
| | | NL 7116919 A | 13-06-1972 |
| US 3812107 A | 21-05-1974 | BE 782943 A1 | 03-11-1972 |
| | | CH 578649 B5 | 13-08-1976 |
| | | CH 651972 D | 13-02-1976 |
| | | DE 2122038 A1 | 23-11-1972 |
| | | FR 2135305 A5 | 15-12-1972 |
| | | GB 1362067 A | 30-07-1974 |
| | | IT 965050 B | 31-01-1974 |
| | | JP 56045947 B | 29-10-1981 |
| | | NL 7205961 A | 07-11-1972 |
| GB 769163 A | 27-02-1957 | NL 95080 C | |
| | | NL 197184 A | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0216479 A
- US 3915921 A
- US 4509949 A
- FR 2416723
- US 2798053 A
- US 2923692 A
- US 2528378 A
- US 2781354 A
- WO 9515144 A
- WO 9501772 A
- EP 714954 A
- FR 2822696
- FR 2825702
- FR 2825625
- FR 2822698
- FR 2822693
- FR 2822694
- FR 2829926
- FR 2807650
- WO 02078660 A
- WO 02100834 A
- WO 02100369 A
- FR 2844269
- GB 1026978 A
- GB 1153196 A
- DE 2359399
- JP 63169571 A
- JP 05163124 A
- EP 0770375 A
- WO 9615765 A
- FR 2750048 A
- DE 3843892
- DE 4133957
- WO 9408969 A
- WO 9408970 A
- FR 2733749 A
- DE 19543988
- FR 2586913

### Littérature non-brevet citée dans la description

- Encyclopedia of Chemical Technology. Kirk-Othmer, 1982, vol. 3, 896-900
- ENCYCLOPEDIA OF CHEMICAL TECHNOLOGY. vol. 15, 439-458
- Polymers in Nature. John Wiley & Sons, 1980, vol. 6, 240-328
- l'Industrial Gums - Polysaccharides and their Dérivatives. Academic Press Inc,
- Handbook of Surfactants. Blackie & Son, 1991, 116-178
- CTFA. 1993